# EUROPÄISCHE PATENTSCHRIFT

(11) **EP 1 349 547 B1**
(45) Veröffentlichungstag und Bekanntmachung des Hinweises auf die Patenterteilung: **13.10.2004**
(21) Anmeldenummer: 01986417.2
(22) Anmeldetag: 17.12.2001
(51) Int. Cl.: A61K 31/195, A61K 49/00, A61P 9/00, A61K 41/00

(54) **VERWENDUNG VON 5-AMINOLÄVULINSÄURE ZUR RESTENOSE-PROPHYLAXE**
USE OF 5-AMINOLEVULINIC ACID FOR THE PROPHYLAXIS OF RESTENOSIS
UTILISATION D'ACIDE 5-AMINOLEVULINIQUE POUR ASSURER LA PROPHYLAXIE DE LA RESTENOSE

(30) Priorität: 18.12.2000 DE 10063076
(43) Veröffentlichungstag der Anmeldung: 08.10.2003
(73) Patentinhaber: Photonamic GmbH & Co. KG, 20354 Hamburg (DE)
(72) Erfinder: DETTMANN, Ralph, 22159 Hamburg (DE)
(74) Vertreter: UEXKÜLL & STOLBERG
(86) Internationale Anmeldenummer: PCT/EP2001/014884
(87) Internationale Veröffentlichungsnummer: WO 2002/049635

(56) Entgegenhaltungen:
- DE-A- 19 852 245
- JENKINS M P ET AL: "Clinical study of adjuvant photodynamic therapy to reduce restenosis following femoral angioplasty." THE BRITISH JOURNAL OF SURGERY. ENGLAND OCT 1999, Bd. 86, Nr. 10, Oktober 1999 (1999-10), Seiten 1258-1263, XP002202725 ISSN: 0007-1323 in der Anmeldung erwähnt
- NYAMEKYE ISAAC ET AL: "Photodynamic therapy of normal and balloon-injured rat carotid arteries using 5-amino-levulinic acid." CIRCULATION, Bd. 91, Nr. 2, 1995, Seiten 417-425, XP002202726 ISSN: 0009-7322
- MICHAEL JENKINS, GIOVANNI BUONACCORSI, A MACROBERT, C BISHOP, STEPHEN G BROWN, J MACEWAN: "pharmacokinetics and efficacity of 5-aminolevulinic acid for endovascular photodynamic therapy in a swine model" PROCEEDINGS OF SPIE - THE INTERNATIONAL SOCIETY FOR OPTICAL ENGINEERING, Bd. 3245, Januar 1998 (1998-01), Seiten 20-27, XP001080578
- M JENKINS, G BUONACCORSI, A MACROBERT, C C R BISHOP, S G BROWN, J R MCEWANS: "intra-arterial photodynamic therapy using ALA-5 in a swine model" EUR J ENDOVASC SURG , Bd. 16, 1998, Seiten 284-291, XP001079614
- JENKINS M P ET AL: "Reduction in the response to coronary and iliac artery injury with photodynamic therapy using 5-aminolaevulinic acid." CARDIOVASCULAR RESEARCH. NETHERLANDS 14 JAN 2000, Bd. 45, Nr. 2, 14. Januar 2000 (2000-01-14), Seiten 478-485, XP002202727 ISSN: 0008-6363
- GABELER E E E ET AL: "ENDOVASCULAR PHOTODYNAMIC THERAPY INHIBITS INTIMAL HYPERPLASIA AFTER PTA IN A RAT MODEL" PROCEEDINGS OF THE SPIE, SPIE, BELLINGHAM, VA, US, Bd. 4156, 2001, Seiten 165-174, XP001016355

## Beschreibung

Die vorliegende Erfindung betrifft die Verwendung von 5-Aminolävulinsäure zur Restenose-Prophylaxe bei photodynamischer Therapie unter Anwendung subletaler Lichtdosen.

Die perkutane transluminale Angioplastie stellt ein etabliertes Behandlungsverfahren dar, koronare und periphere arteriosklerotische Gefäßstenosen zu beseitigen.

Weltweit werden jährlich mehr als 800 000 koronare Interventionen durchgeführt, ca. 150 000 davon in Deutschland, mit dem Ziel, die Prävalenz der stummen oder symptomatischen (Belastungs-) Ischämie zu reduzieren oder zu beseitigen und die Belastungskapazität zu verbessern [E. Bruckenberger, Herzbericht 1998, 55-58; Unverdorben et al., Dtsch. Med. Wschr. 123 (1998) 1046-1051]. Hinzu kommen allein in Deutschland jährlich mind. 15-20 000 periphere Angioplastien [Deutsche Gefäßliga, persönliche Mitteilungen].

Die primären Behandlungserfolge von Koronarinterventionen liegen bei 90-95% [Fuster et al., Thrombosis and Hemostasis 74 (1995) 552-559]. Limitiert wird die Effektivität des Verfahrens dennoch durch die hohe Restenosierungsrate, die abhängig von der Art der Intervention und von Patientencharakteristika bei 15-47% innerhalb der ersten 6 Monate nach der Angioplastie liegt, und die eine erneute Intervention erforderlich macht. [Holmes et al., Am. J. Cardiol. 53 (1984) 77C-81C; Leimgruber et al., Circulation 73 (1986) 710-717; Mabin et al., Circulation 71 (1985) 754-760; Vandormeal et al., Am. J. Cardiol. 10 (1987) 246-252].

Eine komplexe, bisher nur teilweise aufgeklärte Kaskade von Ereignissen führt zur erneuten Lumeneinengung nach dem primären Eingriff. Thrombusformation an der interventionsbedingten Verletzungsstelle, Neointimabildung, Folge der Migration und Proliferation glatter Gefäßmuskelzellen, die Produktion extrazellulärer Matrix sowie ein negatives Remodelling der Gefäßwand tragen in unterschiedlichem Ausmaß zur Restenoseentwicklung bei [Kastrati et al., Herz 25 (2000) 34-46; Fuster et al., Thrombosis and Hemostasis 74 (1995) 552-559].

Durch die Implantation metallischer Gefäßstützen (Stents) konnten die Restenoseraten im Vergleich zur alleinigen Angioplastie zwar gesenkt werden [Fishman et al., N. Engl. J. Med. 331 (1994) 496-501; Serruys et al., N. Engl. J. Med. 331 (1994) 489-495], berücksichtigt werden muß aber, daß nach Dilatation einer Stent-Stenose die Lumenreduktion um 15% größer ist als nach Stentimplantation. Die angiographische Rezidivrate ist mit 30-85% nach Stent-Dilatation größer als nach Dilatation einer Primärstenose [Reimer et al., J. Am. Coll. Cardiol. 30 (1997) 186-192; Topaz et al., Cath. Cardiovasc. Diagn. 37 (1996) 293-299].

Die Behandlungsalternativen Rotablation, Atherektomie und medikamentöse Therapieansätze haben bisher keine entscheidenden Vorteile bei der Restenoseverhinderung gezeigt [Unverdorben 1998]. Lediglich durch die Brachytherapie mit ionisierenden β-oder γ-Strahlen konnte die Neointimabildung reduziert und eine Lumenvergrößerung nach Angioplastie erzielt werden [Verin et al., Circulation 95 (1997) 1138-1144]. Mittel-und Langzeitergebnisse der Brachytherapie sind bisher nicht bekannt, und vor allem fehlen Daten zum Langzeitrisiko der Behandlung mit ionisierenden Strahlen in dieser Indikation [Jenkins et al., Cardiovascular Research 45 (2000) 478-485].

In den vergangenen Jahren wurde zur Unterdrückung der Restenoseentwicklung die Photodynamische Therapie (PDT) vorgeschlagen, deren wirkmechanismus durch die Zytotoxizität des gebildeten Singulettsauerstoffs gekennzeichnet ist.

In tierexperimentellen Untersuchungen am Schwein wurde eine photodynamische Therapie zur Restenoseprophylaxe mit 5-Aminolävulinsäure (5-ALA) in einer Konzentration von 120mg/kg Körpergewicht durchgeführt. Die Bestrahlung erfolgte mit Rotlicht (λ = 635nm) mit einer Lichtstärke von 50J/cm² (0,037 Watt und 1350 Sekunden - 0,026 Watt und 1950 Sekunden). Im Rahmen einer Pilotstudie mit 7 Patienten, bei der die photodynamische Therapie direkt im Anschluß an eine Dilatation der Femoralarterie erfolgte, wurden 60mg/kg Körpergewicht 5-Aminolävulinsäure zur Sensibilisierung verwendet und die dilatierten Gefäßsegmente mit einer Lichtstärke von ebenfalls 50J/cm² (0,12 Watt, 417 Sekunden) bestrahlt. Keiner der so behandelten Patienten entwickelte in den folgenden-6 Monaten eine Restenose. Alle Patienten blieben nach der Therapie symptomfrei, und auch die hämodynamischen Parameter blieben über den Follow-up-Zeitraum hinweg gegenüber den Eingangsbefunden signifikant gebessert. Die Behandlung blieb in allen Fällen ohne schwere Komplikationen, lediglich bei 2 Patienten traten leichte Nebenwirkungen auf, die spontan zurückgingen [Jenkins et al., Br. J. Surg. 86 (1999) 1258-1263.

In den bisher vorliegenden Arbeiten läßt sich ein zytotoxischer Effekt der photodynamischen Therapie mit 5-Aminolävulinsäure nachweisen. Die Anzahl glatter Muskelzellen der Media läßt sich unter den oben beschriebenen Bedingungen im Tierversuch auf 25% im Vergleich zu unbehandelten Gefäßen reduzieren [Jenkins et al., Eur J Vase Endovasc Surg 16 (1998) 284-291]. Ein zytotoxischer Effekt auf die Myozyten der Media trägt auf diese Weise sicher zur Restenoseverhinderung bei, berücksichtigt werden muß jedoch, daß eine Zerstörung der glatten Muskelzellen der Media den physiologischen Gefäßwandaufbau verändert. Es muß davon ausgegangen werden, daß eine bestrahlungsbedingte Strikturbildung in der Gefäßwand oder die Beeinträchtigung der Stabilität der Gefäßwand durch den Verlust kontraktiler Myozyten den positiven Effekt der Restenoseverhinderung reduzieren.

Da bislang keine Therapieansätze zur Restenose-Prophylaxe zur Verfügung stehen, die ohne bzw. ohne wesentliche Nebenwirkungen sind und bei denen nicht auch gesunde Gefäßwandzellen stark beschädigt werden, ist es Aufgabe der Erfindung, ein neues Behandlungsverfahren bzw. ein pharmazeutisches Präparat zur Verfügung zu stellen, mit dem eine prophylaktische Behandlung ermöglicht wird, die die im Stand der Technik bekannten Nachteile und Nebenwirkungen nicht aufweist.

Im Rahmen der vorliegenden Erfindung wurde nunmehr überraschenderweise festgestellt, daß sich 5-ALA im Gegensatz zu den bisherigen Erfahrungen und Erkenntnissen in ausgezeichneter Weise zur Restenose-Prophylaxe eignet, wenn man die PDT mit subletalen, immunmodulatorisch wirksamen Lichtdosen durchführt.

Kombiniert man 5-Aminolävulinsäure mit niedrigen Lichtdosen, wird keine zytotoxische Wirksamkeit erzielt, und proliferierende Myozyten werden nicht zerstört. Es lassen sich somit die spezifischen, immunkompetenten Reaktionen durch Beeinflussung von Leukozyten und Makrophagen unterdrücken, die für die Auslösung der Proliferationsaktivität der Myozyten verantwortlich sind. Da der initiale Schritt des oben beschriebenen generellen Wundheilungsprozesses durch die photodynamische Therapie unterbrochen wird, ist eine Restenoseprophylaxe ohne zytotoxische Effekte mit 5-Aminolävulinsäure und subletalen Lichtdosen von 10-35J/cm² bestrahlter Fläche möglich.

Gegenstand der vorliegenden Erfindung ist daher die Verwendung von 5-ALA zur Herstellung eines pharmazeutischen Präparats zur Restenose-Prophylaxe bei photodynamischer Therapie mit subletalen Lichtdosen von 10 bis 35 J/cm² bestrahlter Fläche.

Die Lichtintensität liegt vorzugsweise bei etwa 15 J/cm². Die verwendete 5-ALA wird mit pharmazeutisch verträglichen Hilfs- und Trägerstoffen in Form eines pharmazeutischen Präparats formuliert, das geeignet ist, die Substanz in verträglicher Art und weise zu applizieren. Vorzugsweise liegt 5-Aminolävulinsäure bzw. das Präparat in gelöster Form vor, vorzugsweise in einer 5,7%igen Natriumhydrogenphosphat-Pufferlösung. Die 5-Aminolävulinsäure-Konzentration liegt im allgemeinen im Bereich von 1 bis 5 Gew.%, vorzugsweise im Bereich von 1,5-3 Gew.%.

Für eine effektive Behandlung werden dem Patienten 30-40mg/kg Körpergewicht 5-Aminolävulinsäure, vorzugsweise 30mg/kg, verabreicht, und das pharmazeutische Präparat ist daher so formuliert, daß es zur Verabreichung dieser Mengen geeignet ist, d.h. es liegt vorzugsweise in systemisch verabreichbarer Zubereitunsform vor. Die Bestrahlung erfolgt anschließend innerhalb von 2-4 Stunden für 100 bis 300 Sekunden im subletalen Lichtdosisbereich (10 bis 35 J/cm²) bei einer Wellenlänge von 635nm.

Wie im Rahmen der vorliegenden Erfindung festgestellt wurde, wird durch Applikation von 30-40mg 5-Aminolävulinsäure pro kg Körpergewicht und Anwendung von Lichtdosen im Bereich von 10-35 J/cm² (bei 635 nm) kein zytotoxischer, sondern ein immunmodulatorischer Effekt erzielt.

Da der immunmodulatorische Effekt der photodynamischen Therapie mit 5-Aminolävulinsäure zur Restenoseprophylaxe führt, ohne daß es zu den beschriebenen nachteiligen zytotoxischen Effekten kommt, und die damit verbundenen Behandlungserfolge nicht vorhersehbar waren, wird durch die vorliegende Erfindung erstmals eine wirksame Therapie zur Restenoseprophylaxe, d.h. ein im Rahmen der photodynamischen Therapie mit niedrigen, subletalen Lichtdosen wirksames Arzneimittel, zur Verfügung gestellt.

Die Erfindung wird nachfolgend anhand von Beispielen erläutert.

### Beispiele

Aus bereits publizierten Untersuchungen geht hervor, daß eine photodynamische Therapie mit 5-Aminolävulinsäure und Lichtdosen von 50J/cm² zu einer Verminderung der Zahl der glatten Muskelzellen der Media führt, die Wirkung also auf einem zytotoxischen Effekt beruht (Jenkins et al., Eur J Vasc Surg 16 (1998) 284-291).

Die erfindungsgemäß erzielte Restenoseunterdrückung durch die photodynamische Therapie mit 5-Aminolävulinsäure und subletalen Lichtdosen wurde am Tierversuchsmodell erstmals nachgewiesen.

Durch die Implantation von metallischen Gefäßstützen (Stents) wurden in koronaren und peripheren Arterien von Schweinen Verletzungen induziert. Durch eine solche Verletzungsinduktion wird die Bildung einer Neointima induziert, die, wie oben beschrieben, durch die Proliferation und Migration glatter Muskelzellen verursacht wird.

Messungen 14 Tage nach Verletzungsinduktion zeigten unbehandelt eine Neointimabildung, d.h. eine Gefäßwandverdickung an der Verletzungsstelle, von im Mittel 2,09mm². Eine im Anschluß an die Verletzung durchgeführte photodynamische Therapie mit 5-Aminolävulinsäure und Lichtdosen von 25J/cm² führte zu einer Reduktion der Neointimadicke von im Mittel 87% auf 0,28 mm².

Die photodynamische Therapie wurde durchgeführt mit:
- **5-Aminolävulinsäure:**: intravenöse Applikation einer 1,5%igen Lösung in 5,7%igem Natriummonohydrogenphosphat-Puffer
- **Dosierung:**: 30mg/kg Körpergewicht
- **Licht:**: Rotlicht der Wellenlänge 635nm
- **Lichtdosis:**: 25J/cm². (0,1 Watt über 250 Sekunden)

Bei der in der Versuchsreihe verwendeten Lichtdosis von 25J/cm² konnten histologisch keine Nekrosen der Gefäßwandstrukturen nachgewiesen werden. Die beobachteten Effekte der Restenoseunterdrückung können demnach im Bereich subletaler, nicht zytotoxisch wirksamer Bestrahlungsdosen erzielt werden.

## Patentansprüche

1. Verwendung von 5-Aminolävulinsäure (ALA) zur Herstellung eines pharmazeutischen Präparates zur Restenose-Prophylaxe durch photodynamische Therapie mit Rotlicht (λ = 635nm) bei subletalen Lichtdosen von 10 bis 35 J/cm² bestrahlter Fläche.

2. Verwendung nach Anspruch 1 bei Lichtdosen von 15 J/cm².

3. Verwendung nach Anspruch 1 oder 2, **dadurch gekennzeichnet, daß** das pharmazeutische Präparat in systemisch verabreichbarer Zubereitungsform vorliegt.

4. Verwendung nach Anspruch 3, **dadurch gekennzeichnet, daß** das pharmazeutische Präparat so formuliert ist, daß es zur Applikation von 30-40mg/kg Körpergewicht 5-Aminolävulinsäure geeignet ist.

5. Verwendung nach Anspruch 4, **dadurch gekennzeichnet, daß** es zur Applikation von 30mg/kg Körpergewicht 5-Aminolävulinsäure geeignet ist.

## Claims

1. Use of 5-aminolaevulinic acid (ALA) for producing a pharmaceutical product for restenosis prophylaxis by photodynamic therapy with red light (λ = 635nm) with sublethal light doses of 10 to 35 J/cm² of irritated area.

2. Use according to Claim 1 with light doses of 15 J/cm².

3. Use according to Claim 1 or 2, **characterized in that** the pharmaceutical product is in the form of a formulation which can be administered systemically.

4. Use according to Claim 3, **characterized in that** the pharmaceutical product is formulated such that it is suitable for administering 30-40 mg/kg body weight of 5-aminolaevulinic acid.

5. Use according to Claim 4, **characterized in that** it is suitable for administering 30 mg/kg body weight of 5-aminolaevulinic acid.

## Revendications

1. Utilisation d'acide 5-aminolévulinique (ALA) dans la fabrication d'une préparation pharmaceutique en vue du traitement prophylactique de la resténose par thérapie photodynamique avec de la lumière rouge (λ = 635 nm) à des doses de rayonnement lumineux sublétales de 10 à 35 J/cm² de surface irradiée.

2. Utilisation selon la revendication 1 à des doses de rayonnement lumineux de 15 J/cm².

3. Utilisation selon la revendication 1 ou 2, **caractérisée en ce que** la préparation pharmaceutique existe sous une forme de préparation administrable par voie systémique.

4. Utilisation selon la revendication 3, **caractérisée en ce que** la préparation pharmaceutique est formulée de telle sorte qu'elle est appropriée à l'application de 30 à 40 mg d'acide 5-aminolévulinique par kg de poids du corps.

5. Utilisation selon la revendication 4, **caractérisée en ce qu'**elle est appropriée à l'application de 30 mg d'acide 5-aminolévulinique par kg de poids du corps.
